Europäisches Patentamt

European Patent Office

Office européen des brevets

(11) Numéro de publication: **0 178 990**
**A1**

# (12) DEMANDE DE BREVET EUROPEEN

(21) Numéro de dépôt: **85401968.4**

(22) Date de dépôt: **09.10.85**

(51) Int. Cl.⁴: **A 61 B 5/04**

(30) Priorité: **11.10.84 FR 8415594**

(43) Date de publication de la demande:
**23.04.86 Bulletin 86/17**

(84) Etats contractants désignés:
**AT BE CH DE GB IT LI LU NL SE**

(71) Demandeur: **Ascher, Gilles**
**20bis, Boulevard du Général Leclerc**
**F-92200 Neuilly(FR)**

(72) Inventeur: **Ascher, Gilles**
**20bis, Boulevard du Général Leclerc**
**F-92200 Neuilly(FR)**

(74) Mandataire: **Rodhain, Claude et al,**
**Cabinet Claude Rodhain 30, rue la Boétie**
**F-75008 Paris(FR)**

(54) **Enregistreur portatif d'électrocardiogrammes.**

(57) Un enregistreur portatif d'électrocardiogrammes est constitué d'un boîtier (1) renfermant un dispositif de traitement numérique des signaux prélevés par au moins deux électrodes (5, 6, 9, 10) en contact avec la peau du patient.

L'une des faces d'application (3) du boîtier (1) comprend des électrodes (5, 6) en saillie formant des protubérances par rapport à ladite face (3) es électrodes (5, 6) comportent des cavités d'accouplement mécanique et de connexion électrique (7, 8) dont la forme est la form complémentaire de celle des tétons des électrodes usuelles (9, 11) d'électrocardiogrammes fixées par adhésion sur la peau du patient, de sorte que ledit boîtier peut être appliqué soit directement sur la peau du patient, soit indirectement par l'intermédiaire d'électrodes uselles.

Application notamment au diagnostic rapide de maladies cardiaques.

FIG.2

"Enregistreur portatif d'électrocardiogrammes"

La présente invention concerne un enregistreur portatif d'électrocardiogrammes, c'est-à-dire un appareil essentiellement constitué d'un boîtier renfermant un dispositif de traitement numérique des signaux prélevés par au moins deux électrodes en contact avec la peau du patient lu et analysé ultérieurement par un médecin.

On a déjà conçu des appareils de ce type qui comporte des électrodes de prélèvement constituées par des petites plaques conductrices disposées dans une des faces de l'appareil. Ces appareils sont destinés à enregistrer quelques électrocardiogrammes lors de l'apparition d'un malaise, le patient mettant alors les électrodes en contact avec sa peau, par exemple en serrant l'appareil entre ses deux mains lorsqu'il ressent un malaise. Il est également possible d'effectuer un enregistrement péricordial en portant les électrodes sur le thorax. Toutefois dans ce cas la qualité du contact peut être aléatoire compte tenu du profil du buste du patient et des variations de pression inévitables exercées (patient ou tierce personne) lors de l'application de l'appareil. Par ailleurs, une telle situation n'est concevable que pour des enregistrements assez brefs dans la mesure où elle suppose le maintien en pression de l'appareil sur le thorax.

De plus, il est désirable que l'appareil puisse être utilisé "en temps réel", c'est-à-dire par exemple dans le cabinet d'un médecin; dans ce cas il est nécessaire de pouvoir visualiser directement sur l'appareil les signaux obtenus afin d'éviter de passer par l'intermédiaire d'un appareil extérieur qui lit la mémoire et restitue les signaux d'électrocardiogrammes.

La présente invention a pour objet un enregistreur du type précité qui permet d'obtenir un bon contact des électrodes lors de la prise d'électrocardiogrammes dans la zone péricordiale, de pouvoir maintenir l'enregistreur en position de fonctionnement et de l'utiliser en temps réel.

L'enregistreur selon l'invention est notamment remarquable en ce qu'au moins une des faces d'application du boîtier comprend des électrodes en saillies formant des protubérances par rapport à ladite face, ces électrodes comportant des cavités d'accouplement mécanique et de connexion électrique dont la forme est la forme complémentaire de celle des tétons des électrodes usuelles d'électrocardiogrammes fixées par adhésion sur la peau du patient, de sorte que ledit boîtier peut être appliqué soit directement sur la peau du patient, soit indirectement par l'intermédiaire d'électrodes usuelles. L'invention permet donc de disposer d'un appareil qui peut être utilisé rapidement en le portant sur la poitrine d'un patient et qui peut également être fixé de manière permanente sur un patient en utilisant des électrodes usuelles du type jetable par conséquent de manière simple et peu couteuse.

Selon une autre caractéristique de l'invention, l'enregistreur comporte un dispositif de visualisation disposé sur la face opposée à la face d'application des électrodes et un microprocesseur commandant ledit dispositif de visualisation. La présence d'un dispositif de visualisation permet de faire fonctionner l'enregistreur en "temps réel" c'est-à-dire que l'on peut suivre la forme des signaux d'électrocardiogrammes enregistrés au fur et à mesure de leur enregistrement. Un tel dispositif permet en particulier de faire un premier diagnostic lors d'une visite à un malade.

Avantageusement, le dispositif de visualisation comporte un dispositif d'arrêt sur image, ce qui permet une analyse plus précise des signaux enregistrés.

L'enregisteur selon l'invention comporte avantageusement une mémoire dans laquelle les signaux d'électrocardiogrammes enregistrés sont mémorisés sous forme numérique, ce qui permet d'effectuer une surveillance plus complète d'un malade.

D'autres caractéristiques et avantages de l'invention ressortiront de la description qui suit, faite à titre illustratif en se référant aux dessins sur lesquels :

- la fig. 1 est une vue de côté d'un enregistreur conforme à la présente invention en position sur un patient,

- la fig. 2 représente l'enregistreur de la figure 1 fixé sur un patient au moyen d'électrodes usuelles du type jetable,

- la fig. 3 est une vue de dessus de l'enregistreur de la figure 1.

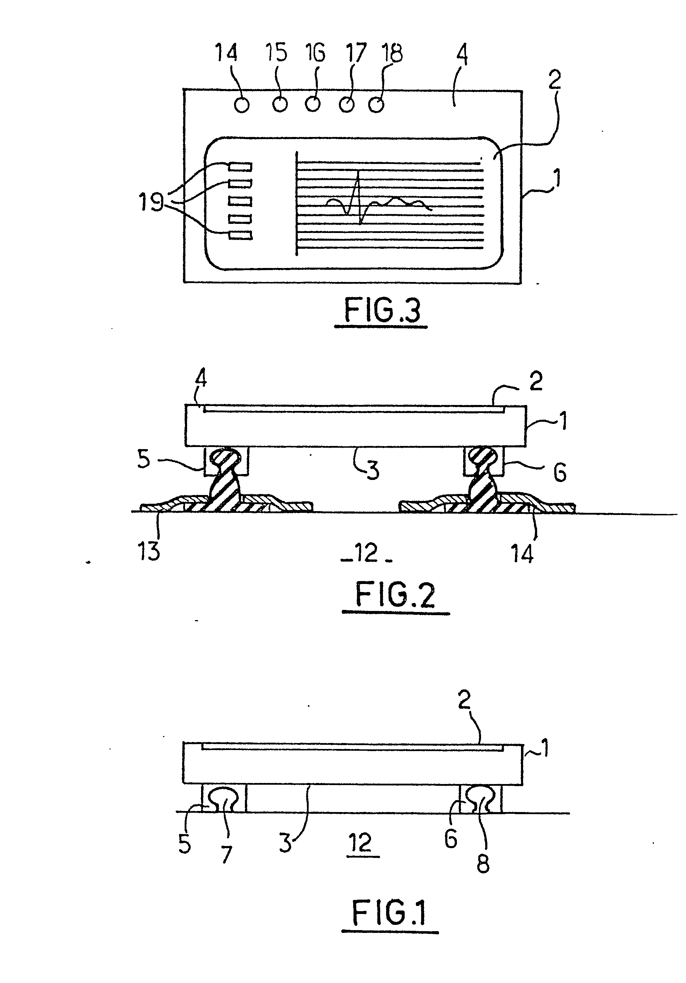

On voit sur les figures 1 à 3 un enregistreur qui est constitué d'un boîtier 1 de faibles dimensions dont l'une des faces porte un dispositif de visualisation 2 constitué par exemple par un écran à cristaux liquides; sur la face d'application 3 qui est opposée à la face 4 portant l'écran 2 sont disposées au moins deux électrodes 5 ou 6, par exemple trois électrodes dont une électrode constituant l'électrode de référence. Les électrodes 5 et 6 font saillie de la face 3 et forment des protubérances sur cette face Elles comportent des cavités 7 et 8 débouchant du côté opposé à la face d'application et dont la forme correspond à la forme des tétons de fixation des électrodes usuelles d'électrocardiogrammes du type jetable. Grâce à cette disposition, des électrodes usuelles 9 et 11 peuvent être fixées sur les électrodes 5 et 6 du boîtier qui peut alors être fixé en permanence sur la peau 12 du patient comme représenté sur la figure 2. Du fait du faible poids du boîtier 1, deux ou trois électrodes usuelles suffisent pour maintenir le boîtier sur le corps du patient même si celui-ci se déplace. En effet, les électrodes usuelles d'électrocardiogrammes 9 et 11 comportent une pastille adhésive 13 qui est collée sur la peau 12 du patient.

L'enregistreur selon l'invention comprend un microprocesseur qui est logé dans le boîtier 1 et qui commande le fonctionnement du dispositif de visualisation ainsi que le traitement des signaux d'électrocardiogrammes prélevés.

Avantageusement, l'enregistreur comporte également une mémoire dans laquelle sont mémorisés sous forme numérique les signaux d'électrocardiogrammes prélevés au moyen des électrodes 5 et 6.

Une commande 14 permet d'obtenir l'enregistrement des électrocardiogrammes prélevés, la mémorisation se faisant en permanence tant que cette commande 14 est actionnée, la mémorisation se faisant selon le mode "premier entré , premier sorti".

Un interrupteur 15 à deux positions stables commande la marche et l'arrêt de l'enregistreur selon l'invention. Pendant le fonctionnement, le dispositif de visualisation 2 affiche les formes de signaux recueillis; la vitesse de défilement peut être réglée au moyen d'un organe de commande 16 et un autre organe de commande 17 permet d'obtenir un arrêt sur l'image c'est-à-dire l'affichage d'une image fixe.

Lorsque l'enregistreur comporte une mémoire il est possible d'afficher le contenu de la mémoire et pour éviter des manipulations compliquées, l'enregistreur comporte un organe de commande 18 qui commande la lecture en avant ou en arrière de la mémoire.

Avantageusement, le dispositif électronique de traitement comporte un dispositif de calcul de la fréquence cardiaque qui calcul ladite fréquence par exemple en se basant sur la détection de trois fronts QRS de trois électrocardiogrammes successifs. Dans ce cas, le dispositif de visualisation 2 affiche en plus la fréquence cardiaque du sujet et il peut comporter un élément clignotant à la fréquence du rythme cardiaque de l'électrocardiogramme prélevé.

L'enregistreur selon l'invention peut comporter des connexions de sortie à une imprimante ou à un appareil d'électrocardiogramme classique ou encore ces deux possibilités.

Selon une autre variante de l'enregistreur selon l'invention, le dispositif électronique de traitement effectue une analyse des signaux d'électrocardiogrammes prélevés et calcule leurs différents paramètres qui sont alors affichés simultanément sur le dispositif de visualisation 2; on voit sur la figure 3 l'écran 2 qui affiche d'une part un électrocardiogramme et d'autre part en des emplacements 19 les différents paramètres de l'électrocardiogramme ainsi que la fréquence du rythme cardiaque.

En outre, l'enregistreur selon l'invention peut comporter un dispositif de surveillance du rythme cardiaque comportant une alarme sonore et/ou visuelle qui se déclenche lorsque le ryth.me cardiaque dépasse des valeurs limites supérieure et inférieure réglables.

L'enregistreur qui vient d'être décrit peut être utilisé de différentes manières Dans une première version, comportant essentiellement le dispositif de visualisation avec les différentes commandes et avec l'affichage de la fréquence cardiaque, l'enregistreur peut être utilisé par des médecins généralistes lors de leur visite ou par des médecins de service d'urgence; dans ce cas l'appareil est utilisé sans électrode jetable, le médecin l'applique sur la poitrine du patient et il peut observer aussitôt la forme des électrocardiogrammes et connaître la fréquence cardiaque, ce qui lui permet de
détecter rapidement la possibilité d'une déficience cardiaque et
d'orienter aussitôt le malade vers un service disposant de matériel
de diagnostic cardiologique.

Selon une autre utilisation, dans une version comportant
une mémoire, l'enregistreur selon l'invention peut être utilisée dans
le cabinet du praticien, par exemple pour la surveillance du fonctionnement d'un stimulateur cardiaque; dans ce cas, la mémoire permet
d'examiner soigneusement pendant tout le temps nécessaire la forme
de l'électrocardiogramme soit directement sur l'appareil soit sur
un appareil d'analyse électrocardiogrammes extérieur en utilisant
la connexion prévue à cet effet. Dans le cas où l'on effectue la
connexion à une imprimante, le programme du dispositif électronique
de traitement de l'enregistreur est prévu pour que l'imprimante imprime un électrocardiogramme sous la forme d'un électrocardiogramme
classique, c'est-à-dire avec des axes de coordonnées et des carreaux
donnant immédiatement la valeur des signaux enregistrés. Dans cette
immédiatement la valeur des signaux enregistrés. Dans cette utilisation, l'enregistreur peut être appliqué directement ou par le biais
d'électrodes classiques du type jetable. En outre, il est possible
toujours dans le cadre de la surveillance d'un stimulateur de visualiser la forme des impulsions délivrées par la stimulation et d'opérer une analyse oscilloscopique, comprenant la visualisation de l'impulsion et l'affichage des principaux paramètres :
- largeur d'impulsion
- fréquence et intervalle de stimulation
- délai auriculo-ventriculaire

L'enregistreur selon l'invention peut également
être utilisé comme appareil de surveillance (monitor) d'un patient,
par exemple lors de petites interventions (en raccordant l'appareil
à des électrodes posées sur le patient grâce à un câble de liaison)
pour lesquelles de praticien veut opérer sans aucun risque ou également pour le transport de malades vers un hôpital ou à l'intérieur de
celui-ci (dans cette utilisation, l'enregistreur est placé en permanence

0178990

sur le patient et l'on utilise donc les électrodes de type jetable
pour la fixation de l'enregistreur sur le patient). L'enregistreur,
selon l'invention, constitue un appareil de surveillance portatif
qui affiche en permanence la forme des électrocardiogrammes du patient et qui donne l'alarme lorsque la fréquence cardiaque atteint
un seuil prédéterminé.

REVENDICATIONS

1°) Enregistreur portatif d'électrocardiogrammes constitué d'un boîtier (1) renfermant un dispositif de traitement numérique des signaux prélevés par au moins deux électrodes (5,6,9, 10) en contact avec la peau du patient, caractérisé en ce que l'une des faces d'application (3) comprend des électrodes (5,6) en saillie formant des protubérances par rapport à ladite face (3), ces électrodes (5,6) comportant des cavités d'accouplement mécanique et de connexion électrique dont la forme est la forme complémentaire de celle de tétons d'électrodes (9,11) d'électrocardiogramme fixées par adhésion sur la peau du patient, de sorte que ledit boîtier peut être appliqué soit directement sur la peau du patient, soit indirectement par l'intermédiaire d'électrodes dites jetables.

2°) Enregistreur selon la revendication 1, caractérisé en ce que le dispositif d'accouplement mécanique et électrique est compatible avec les électrodes jetables usuelles.

3°) Enregistreur selon l'une des revendications 2 ou 3, caractérisé en ce que le dispositif de visualisation est constitué par un écran à cristaux liquides.

4°) Enregistreur selon l'une des revendications 2 ou 3, caractérisé en ce que le dispositif de visualisation (2) comporte un dispositif d'arrêt sur image.

5°) Enregistreur selon l'une quelconque des revendications 1 à 4, caractérisé en ce que le micro-processeur effectue une analyse des signaux prélevés et en ce que le dispositif de visualisation (2) affiche le résultat de cette analyse en même temps que le signal d'électrocardiogramme.

6°) Enregistreur selon l'une quelconque des revendications 1 à 5, caractérisé en ce que le micro-processeur effectue le calcul de la fréquence cardiaque et en ce que le dispositif de visualisation (2) affiche la valeur calculée du rythme cardiaque.

7°) Enregistreur selon la revendication 6, caractérisé en ce qu'il comporte un dispositif d'alarme sonore et/ou visuelle actionné lorsque le rythme cardiaque dépasse un seuil réglable.

8°) Enregistreur selon la revendication 5, caractérisé en ce que le dispositif de visualisation (2) affiche la largeur d'impulsion, la fréquence et l'intervalle de stimulation et le délai auriculo-ventriculaire lorsqu'il est utilisé pour la surveillance d'un stimulateur cardiaque.

0178990

14  15  16  17  18      4      2

19                              1

## FIG.3

4                2
                 1

5      3      6

13        _12_        14

## FIG.2

2
1

5  7    3    12    6
                   8

## FIG.1

Office européen
des brevets

**RAPPORT DE RECHERCHE EUROPEENNE**

EP  85 40 1968

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-3 848 582  (D.L. MILANI et al.)<br>* Résumé;  colonne  1, lignes 60-65;  colonne  2, lignes 54-65; colonne  4, lignes 14-22; figures 1,2,7 * | 1 | A 61 B   5/04 |
| A | | 6 | |
| Y | US-A-4 350 164  (J.L. ALLAIN Jr.)<br>* Résumé;  colonne  2, lignes 19-38;  colonne  3, lignes 44-52; colonne  3, ligne 67 - colonne 4, ligne  14;  colonne  6, lignes 27-40;  colonne  8, lignes 17-26; figures 3,4,7 * | 1 | |
| A | PATENTS ABSTRACTS OF JAPAN, vol. 6, no. 66 (P-112)[944], 27 avril 1982; & JP - A - 57 8418 (RICOH TOKEI K.K.) 16-01-1982<br>* En entier * | 1,3,5, 6 | A 61 B |
| A | US-A-4 094 310  (R.A. McEACHERN et al.)<br>* Résumé;  colonne  3, lignes 53-68;  colonne  5, lignes 51-61; colonne  8, lignes 20-25; colonne 10, lignes 11-32; figures 1-3 * | 4,8 | |

DOMAINES TECHNIQUES RECHERCHES (Int. Cl.4)

---   -/-

Le présent rapport de recherche a été établi pour toutes les revendications

| Lieu de la recherche | Date d'achèvement de la recherche | Examinateur |
|---|---|---|
| LA HAYE | 08-01-1986 | RIEB K.D. |

**0178990**
Numero de la demande

**RAPPORT DE RECHERCHE EUROPEENNE**

Office européen
des brevets

EP 85 40 1968

Page 2

| Catégorie | Citation du document avec indication, en cas de besoin, des parties pertinentes | Revendication concernée | CLASSEMENT DE LA DEMANDE (Int Cl 4) |
|---|---|---|---|
| A | DE-A-2 646 866 (D. NORDMEIER) <br><br> * Page 1, revendications 1,3; page 2, revendications 4,7; page 5, lignes 9-33; page 8, lignes 1-12; page 9, lignes 1-9; figure 1 * | 1,3,5, 6 | |
| A | DE-A-2 750 033 (H.P. MEDIZINTECHNIK) <br> * Pages 3,4; revendications 9,10,12; page 6, lignes 13-25; page 8, lignes 8-31; page 13, lignes 25-35; figure 1 * | 4,6,7 | |
| A | GB-A-2 060 174 (MEDRAD, INC.) <br> * Abrégé; page 1, lignes 42-62,90-128; page 2, ligns 57-82; page 3, lignes 86-100,114-125; page 9, lignes 104-126; figure 1 * | 1,4-8 | DOMAINES TECHNIQUES RECHERCHES (Int. Cl.4) |

Le présent rapport de recherche a été établi pour toutes les revendications

| Lieu de la recherche <br> LA HAYE | Date d'achèvement de la recherche <br> 08-01-1986 | Examinateur <br> RIEB K.D. |
|---|---|---|